# EUROPEAN PATENT APPLICATION

(11) **EP 4 075 443 A1**
(43) Date of publication of application: **19.10.2022**
(21) Application number: 19955245.6
(22) Date of filing: 12.12.2019
(51) Int. Cl.: G16H 50/20, G16H 50/50, G16H 50/30, G16H 70/60, G16H 80/00

(54) **HEALTH PREDICTION SYSTEM AND METHOD USING ORAL MICROORGANISM ANALYSIS DEVICE**

(30) Priority: 06.12.2019 KR 20190161760
(71) Applicant: Clinomics Inc., Ulsan 44919 (KR)
(72) Inventor: BHAK, Jong Hwa, Ulju-gun Ulsan 44936 (KR); KIM, Byung Chul, Yongin-si Gyeonggi-do 17082 (KR); CHO, Yun Sung, Yongin-si Gyeonggi-do 16953 (KR)
(74) Representative: Frenkel, Matthias Alexander
(86) International application number: PCT/KR2019/017530
(87) International publication number: WO 2021/112317

(57) **Abstract**

A health prediction system is disclosed. More specifically, the present invention relates to a health prediction system and method using an oral microorganism analysis device, wherein the current health condition of an individual is predicted by collecting and analyzing various microorganisms present in the oral cavity. According to an embodiment of the present invention, a sample is collected from a subject, introduced into a small genome analyzer, and subjected to genome analysis or genotyping, thus having the effect of enabling: the identification of genomic species of microorganisms that come out through the washing of the oral cavity, the brushing of teeth, or gargling; and software, analysis, and follow-up actions utilizing the same.

## Description

### Technical Field

The present invention relates to a health prediction system, and more particularly, to a health prediction system and method using an intraoral microorganism analysis device for predicting an individual's current health state by collecting and analyzing various microorganisms present in the oral cavity.

### Background Art

The oral cavity is a major body portion which numerous pathogenic microorganisms enter and may be said to be the source of diseases, such as colds caused by external microorganisms. In particular, it is microorganisms that cause diseases, such as periodontitis, tooth decay, inflammation and colds.

Microorganisms are organisms that have a size of 0.1 mm or less that exceeds the visible limit of the human eye and make up their body with mainly single cells or mycelium. The microorganisms have a lot of influence on our lives in an invisible area, and these influences exist in a variety of ways, from beneficial to harmful.

Therefore, disease occurrence and health may be predicted by grasping information about microorganisms, such as bacteria, fungi, and viruses inside and outside our body, and various methods for identifying the amount and type of these microorganisms through genetic testing have been proposed.

The health status of the human body is particularly closely related to the intestinal microorganisms and also to the oral organisms. Accordingly, it is possible to predict the state of our body according to the type of microbes (flora) in the oral cavity. Further, it is known that the pattern of the microbiome is related not only to oral health, but also to overall health, such as heart disease.

As an example, periodontitis is estimated to be formed by complex colonization of various oral disease-causing microorganisms (bacteria) living in the oral cavity, and it was identified that these microorganisms co-habitate at the site of oral disease. In particular, it is estimated that diseases, such as periodontitis, are further increased in a situation in which various bacterial species inhabit at the same time. Therefore, it may be said that the distribution of microorganisms living in the oral cavity and periodontal pocket is very important for the diagnosis and treatment of diseases.

### Detailed Description of the Invention

### Technical Problems

The present invention aims to monitor an individual's health status by comparing and analyzing all the microorganisms of the subject, which are scraped through daily or frequent brushing and gargling, using the above-described microorganisms existing in the oral cavity, give advice on health care, and give choices in purchasing a solution.

### Means to Address the Problems

To achieve the foregoing objectives, according to a preferred embodiment of the present invention, a health prediction system using an intraoral microorganism analysis device middle chamber at least one microorganism analysis devices analyzing a sample collected from a subject's oral cavity, performing species identification on the sample, and generating genetic information about a microorganism in the oral cavity according to a result of analysis, and a health-care server collecting the genetic information for each subject from the microorganism analysis device, analyzing the genetic information to obtain a type and population for one or more microorganisms present in the subject's oral cavity, and generating health information about the subject based on a result of the obtaining the type and population.

The microorganism analysis device may include a genome analyzer performing genome analysis on the sample and a user terminal connected with the genome analyzer, receiving a result of the genome analysis, and performing species identification on the microorganism in the oral cavity through comparison with previously stored microbiome data.

The user terminal may include a device connector wiredly or wirelessly connected with the genome analyzer to receive the genetic information, a communication unit connected with the health-care server through an information communication network to perform data communication, data storage storing microbiome data for a plurality of microorganisms for species identification, a genetic information transmitter providing the health-care server with latest genetic information about the subject, generated in response to the result of analysis received from the genome analyzer, and a species identifier performing species identification on one or more microorganisms possessed by the subject through comparison between the result of analysis and the microbiome data and determining the population.

The user terminal may include a health information receiver receiving a health score represented as a numerical value for the subject's health status, from the health-care server through the communication unit.

The health-care server may include a data collector collecting genetic information from one or more user terminals located in areas, through an information communication network, a member information manager performing a membership sign-up, login, and identification procedure for one or more subjects having membership and providing functions of generating, updating, and deleting member information about the subject, a database classifying and storing the member information, genetic information for each subject, and health information and deleting or masking genetic information that has passed a predetermined time after the health information about the subject is generated, a gene analyzer determining the type and population of the microorganism possessed by the subject through the genetic information and estimating and quantifying the subject's current health status based on the type and population of the microorganism to thereby generate the health information, a report generator converting accumulated information about various microbiomes into a report form, and a result provider providing the health information about the subject to each user terminal through the information communication network.

The health-care server may include a predictor predicting each subject's current health status and future health status by comparing the health information accumulated for a predetermined period with the current health information and applying the predicted health statuses to the health information.

The result provider may derive a relative health status between health information about any one subject and health information about a plurality of other subjects and further provide the relative health status to the user terminal.

Further, to achieve the foregoing objectives, according to an embodiment of the present invention, a health prediction method using a microorganism analysis device in an oral cavity may comprise transmitting a result of genome analysis on a sample collected from a subject to a user terminal, by a genome analyzer, generating genetic information by performing species identification on a microorganism in the oral cavity according to the result of genome analysis, by the user terminal, transmitting the genetic information to a health-care server, by the user terminal, classifying genetic information per subject and obtaining a type and population of microorganism possessed by the subject through genetic analysis to generate health information, by the health-care server, and providing the health information to the user terminal, by the health-care server.

The health prediction method may further comprise, after, providing the health information to the user terminal by the health-care server, predicting each subject's current health status and future health status by comparing the health information accumulated for a predetermined period with the current health information and applying the predicted health statuses to the health information, by the health-care server.

The health prediction method may further comprise, after, providing the health information to the user terminal by the health-care server, extracting health information about a plurality of other users than any one subject, deriving a relative health status through comparison with the subject's current health information, and providing the relative health status to the user terminal, by the health-care server.

### Effects of the Invention

According to an embodiment of the present invention, a sample is collected from a subject, introduced into a small genome analyzer, and subjected to genome analysis or genotyping, thus having the effect of enabling: the identification of genomic species of microorganisms that come out through the washing of the oral cavity, the brushing of teeth, or gargling; and software, analysis, and follow-up actions utilizing the same.

### Brief Description of the Drawings

FIG. 1 is a view schematically illustrating an overall structure of a health prediction system using an intraoral microorganism analysis device according to an embodiment of the present invention.
FIG. 2 is a view illustrating a structure of a user terminal of a health prediction system using an intraoral microorganism analysis device according to an embodiment of the present invention.
FIG. 3 is a view illustrating a structure of a health-care server of a health prediction system using an intraoral microorganism analysis device according to an embodiment of the present invention.
FIG. 4 is a view illustrating a health prediction method by a health measurement system according to an embodiment of the present invention.
FIG. 5 is a view illustrating an example of a screen provided by a health prediction system using an intraoral microorganism analysis device according to an embodiment of the present invention.

### Mode to Practice the Invention

Throughout the specification, when an element "includes" another element, the element may further include the other element, rather excluding the other element, unless particularly stated otherwise. Further, the term "unit," "server," or "system" as used herein denote a unit processing one or more functions or operations and be implemented in hardware, software, or a combination thereof.

Although some exemplary embodiments of the disclosure are described herein, the technical spirit or scope of the disclosure are not limited thereto. As used herein, the terms "including," "having," or other similar terms are open-ended transition words that do not exclude any additional or other components when used in the claims and may be comprehensively used in a similar manner to that of "comprising."

Various schemes or methods described herein may be implemented in hardware, software, or a combination thereof. As used herein, the term "unit," "server," or "system" may also be equivalent to a computer-related entity, a hardware component, a software component, or a combination thereof. Each function executed on the program of the present invention may be configured as a module unit and may be recorded in one physical memory or be distributed and recorded between two or more memories and recording media.

In the following description, the phrase "health prediction system using an intraoral microorganism analysis device" may be simply referred to as "health prediction system" or "system" for convenience of description.

Hereinafter, a health prediction system and method using an intraoral microorganism analysis device according to an embodiment of the present invention is described with reference to the drawings.

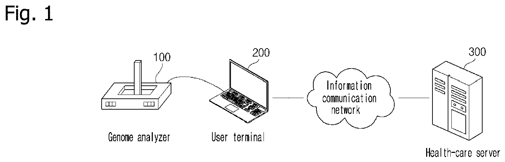

FIG. 1 is a view schematically illustrating an overall structure of a health prediction system using an intraoral microorganism analysis device according to an embodiment of the present invention.

Referring to FIG. 1, a health prediction system using an intraoral microorganism analysis device according to an embodiment of the present invention may include one or more microorganism analysis devices 100 and 200 that analyze a sample collected from the subject's oral cavity, perform species identification, and generate genetic information about the microorganisms in the oral cavity according to the result of analysis and a health measurement system 300 that collects genetic information for each subject from the microorganism analysis devices 100 and 200, analyzes the genetic information to calculate the type and population of one or more microorganisms present in the subject's oral cavity, and generates health information about the subject based on the result of the calculation.

The microorganism analysis devices 100 and 200 may be divided into a genome analyzer 100 for analyzing the sample and a user terminal 200 for performing species identification on the microorganisms.

The genome analyzer 100 is a small electronic device developed to be able to simply discover the activity of microorganisms, which is difficult to discover with the naked eye, through a portable observation means, and may be a device to which various sample analyzing schemes have been applied.

As an example sample analyzing scheme applied to the genome analyzer 100, there may be used a scheme that supplies light to a sample containing microorganisms, which have a fluorescent characteristic of reflecting only a specific wavelength range of light, and collects and images the light reflected by the microorganisms.

As methods for collecting microorganisms in the oral cavity used in the present invention, tissues collected by swabs or paper points from the oral cavity of the subject, mouth rinse, saliva, etc. may be used, but to obtain the result of analysis that better reflects the overall microbial environment in the oral cavity, it is preferable to use not only the saliva on the toothbrush, but also the mouth rinse obtained using water, saline, or gargle.

The genome analyzer 100 may be connected with the user terminal 200 via wired or wireless connection, such as USB or Bluetooth and may transmit the result of sample analyzing to the user terminal 200.

The user terminal 200 may be various types of computing devices, such as a known stationary PC, laptop computer, tablet PC, or personal digital assistant (PDA) and smartphone, and may perform species identification on, and estimate the population of, one or more microorganisms present in the subject's oral cavity, from the result of sample analyzing, and provide genetic information including the result to the health-care server 300 through an information communication network.

In the drawings, the genome analyzer 100 and the user terminal 200 are exemplified as microorganism analysis devices 100 and 200 which are implemented as separate devices and connected to each other. However, without limited thereto, an integrated analysis device may be implemented in such a form that the function of the user terminal 200 is equipped in the genome analyzer 100, according to the designer's intent.

In particular, the user terminal 200 according to an embodiment of the present invention may previously store microorganism data for species identification on microorganisms, and the microorganism data may be downloaded from the health-care server 300. The microorganism data may be standardized genetic reference data which may be compared with the analyzing result to generate genetic information including the type and population of the microorganisms possessed by the subject. The generated genetic information may be provided to the health-care server 300, which is located remotely, through an information communication network.

As the user terminal 200, all types of handheld-based wireless communication devices, such as PCS(Personal Communication System), GSM(Global System for Mobile communications), PDC(Personal Digital Cellular), PHS(Personal Handyphone System), PDA(Personal Digital Assistant), IMT(lnternational Mobile Telecommunication)-2000, CDMA(Code Division Multiple Access)-2000, W-CDMA(W-Code Division Multiple Access), Wibro(Wireless Broadband Internet) terminal, smartphone, smart pad, or tablet PC, and stationary PCs, laptop computers, or other computing devices may be used.

The health-care server 300 may receive genetic information about the subject from the subject, or at the request of one or more user terminals 200 located remotely, through an information communication network and store the genetic information for each subject, calculate a score for disease risk of the species-identified microorganism based on the genetic information, determine disease type, and generate and provide health information about the subject.

Further, the health-care server 300 may convert information about various microbiomes, accumulated in a database, into the form of a report that may be displayed on various media, such as a dedicated application or webpage executed on the user terminal 200, and distribute the report form online. According to this function, a public benefit may be expected of providing the public with a report on the relationship between the microorganisms and diseases, accumulated through the system of the present invention.

Further, the health-care server 300 may compare the past health information accumulated in the database with the current health information, predict variations in the individual's health and the individual's health status, apply the predicted health variations and status to the health information, and provide it to the user terminal 200.

Further, the health-care server 300 may further provide the user terminal with useful information about the type of medicine or toothpaste for dental caries or a suggestion for a visit to the hospital, etc., in response to the type of disease with a high probability of occurrence expected for the subject.

As the health-care server 300, a server computer equipped with a high-performance micro-processor, high-rate and high-volume memory and storage may be used to be able to quickly respond, without delay or errors, to multiple requests and to collect data from multiple user terminals 200.

A user terminal of a health prediction system using an intraoral microorganism analysis device according to an embodiment of the present invention is described below with reference to the drawings.

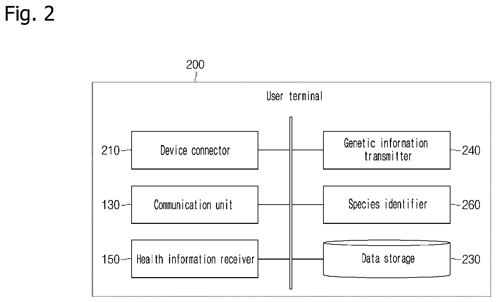

FIG. 2 is a view illustrating a structure of a user terminal 200 of a health prediction system using an intraoral microorganism analysis device according to an embodiment of the present invention. In the following description, each component constituting the user terminal 200 may be implemented as a computer program that is executable by a known micro-processor, recorded in a readable/writable recording medium, and equipped in the user terminal 200.

Referring to FIG. 2, the user terminal 200 of the health prediction system using an intraoral microorganism analysis device according to an embodiment of the present invention may include a device connector 210 that is wiredly or wirelessly connected with the genome analyzer to receive the genetic information, a communication unit 220 that is connected with the health-care server through an information communication network to perform data communication, data storage 230 that stores microbiome data for multiple microorganisms for species identification, a genetic information transmitter 240 that provides the health-care server with latest genetic information about the subject, generated in response to the result of analysis received from the genome analyzer, a species identifier 250 that performs species identification on, and determines the population of, one or more microorganisms possessed by the subject by comparison between the result of analysis and the microbiome data, and a health information receiver 260 that receives health information including a health score, which is a numerical value representing the subject's health status, from the health-care server through the communication unit 220.

The device connector 210 is connected with the genome analyzer in a wired or wireless manner to receive the result of analysis the sample. The device connector 210 may be connected with the genome analyzer in various manners, such as a USB protocol or a Bluetooth protocol.

The communication unit 220 may be connected with the health-care server, which is remotely located, through an information communication network and receive microbiome data for species identification from the connected health-care server. The communication unit 220 may provide the genetic information about the subject, including the result of species identification, to the health-care server and receive health information according to the result of genetic analysis, from the health-care server.

The data storage 230 may store the subject's login information, latest microbiome data for species identification, and the result of species identification using the microbiome. In particular, the microbiome data may be periodically updated as the health-care server distributes a latest version.

The genetic information transmitter 240 may provide the health-care server with the subject's latest genetic information including the population of microorganisms and the result of species identification, derived using the result of analysis received from the genome analyzer, register the subject's data with the health-care server and request health information.

The health information receiver 250 may receive the latest health information derived according to the result of genetic analysis on the subject, from the health-care server. The health-care server may derive health-related information required for the subject and the subject's current health status based on the possessed microorganisms by analyzing the genetic information about the subject, provided from the user terminal, generate and provide health information, and the health information provider 250 may receive the health information through the communication unit 220 and display the health information on the screen of the user terminal 200.

The species identifier 260 may perform species identification on, and determine the population of, the microorganisms possessed by the subject through comparison between the result of sample analyzing transmitted from the genome analyzer and the microbiome data stored in the data storage 230, thereby generating genetic information. The genetic information is used to predict the subject's health status according to the subject's status of possession of microorganisms. The genetic information and a health information request are transmitted through the communication unit 220 and received on the subject's account by the health-care server.

A health-care server of a health prediction system using an intraoral microorganism analysis device according to an embodiment of the present invention is described below with reference to the drawings.

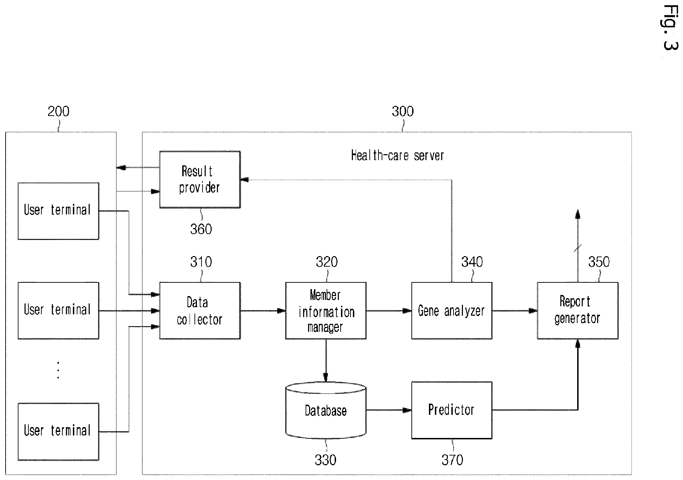

FIG. 3 is a view illustrating a structure of a health-care server of a health prediction system using an intraoral microorganism analysis device according to an embodiment of the present invention.

Referring to FIG. 3, the health-care server 300 according to an embodiment of the present invention may include a data collector 310 that collects genetic information from one or more user terminals 200 in individual areas, through an information communication network, a member information manager 320 that performs a login and identification procedure and provides the functions of generating, updating, and deleting member information about the subject, a database 330 that classifies and stores member information, genetic information about each subject, and health information and deletes or masks genetic information that has passed a predetermined time after health information about the subject is generated, a gene analyzer 340 that determines the type and population of microorganisms possessed by the subject through genetic information and estimates and quantifies the subject's current health status based on the type and population of the microorganisms to generate health information, a report generator 350 that converts accumulated information about various microbiomes into the form of a report, a result provider 360 that provides the health information about the subject to each user terminal through the information communication network, and a predictor 370 that compares health information accumulated for a predetermined period with current health information to thereby predict the current health status and future health status for each subject and applies the predicted health status to health information.

The data collector 310 may frequently collect genetic information including data regarding species identification on the microorganisms analyzed for the subjects from each of multiple user terminals 200 remotely distributed.

The member information manager 320 may identify the subject according to a login request and process of the connected user terminal 200 and store received genetic information in the database 330.

Further, the member information manager 320 may perform a membership procedure and a login/logout procedure on the subjects initially accessing and provide the member managing functions of generating, updating, and deleting member information about the subjects having membership.

The database 330 may store the genetic information received from the user terminal 200, per subject. The genetic information stored in the database 330 may include the type and population of the microorganisms in the subject's oral cavity and, since the genetic information is sensitive personal biometric information, a high-level security policy may be applied thereto.

Further, the database 330 may store various setting values for operating the health-care server.

The gene analyzer 340 may analyze the possibility of developing and type of a specific disease depending on the type and population of the microorganisms possessed by the subject, included in the genetic information about each subject's microorganisms, based on the genetic information, calculates the subject's disease risk level, which is a numerical value, to thereby generate health information. The disease risk level may be represented as a disease score and, together with disease type information, is included in the health information. The health information is provided to the corresponding subject.

Further, if, as a result of the analysis, it is apparent that the microorganism possessed by the subject is pathogenic, the gene analyzer 340 may add advice according to the type of disease to the health information. As an example, in the case of tooth decay, information about a self-diagnosis method and type of toothpaste suitable for tooth decay may be provided, and information about a special clinic may be provided to propose a visit to the clinic.

The report generator 350 may convert various accumulated information about various microbiomes into a report form. Health information may be said to be a measure indicating the current health status of the subject according to whether the individual possesses microorganisms, and separately therefrom, information about various microbiomes may be accumulated in the health-care server 300. The report generator 350 may convert the information accumulated in the health-care server 300 into a report form and distribute it online.

The above-described report may provide the user terminal 200 and other Internet users with various information about diseases related to microorganisms, such as the type of disease caused by each microorganism, prevention and treatment methods and may be generated in the form displayable on a dedicated application or webpage.

The result provider 360 may provide the health information about the subject to each user terminal through the information communication network. The health information may include a disease score calculated based on the microorganisms possessed by the subject, and the subject may previously estimate the possibility of disease occurrence according to the disease score, and prepare for it.

In particular, the result provider 360 may derive relative health statuses through comparison between health information about any one subject and health information about a plurality of other subjects and provide the relative health statuses to the user terminal 200.

The relative health status may be used to determine the circumstance where contagious disease, such as the flue, is prevalent and to grasp a relative position of each subject's current status relative to others, such as whether the current health status is normal or requires caution.

The predictor 370 may generate data that predicts the status and changes in health status of the subject from the past to present by comparing the latest health information and past health information by referring to the database 330 for each subject and may apply the generated data to the health information or provide the generated data, in the form of a report, to the subject.

A health prediction method by a health prediction system using an intraoral microorganism analysis device described above is described below with reference to the drawings.

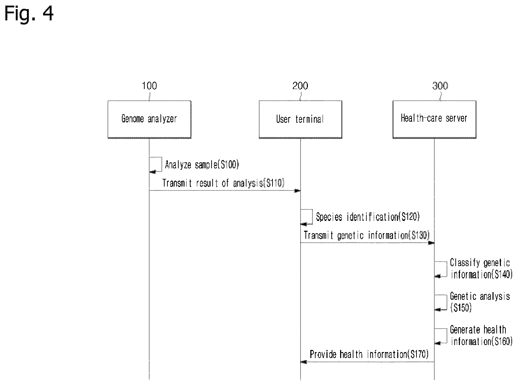

FIG. 4 is a view illustrating a health prediction method by a health measurement system according to an embodiment of the present invention.

Referring to FIG. 4, in a health prediction method by a health measurement system according to an embodiment of the present invention, the genome analyzer 100 performs genome analysis on a sample collected from the subject (S100) and transmits the result of analysis to the user terminal 200 wiredly or wirelessly connected (S110).

Subsequently, the user terminal 200 compares the result of analysis with microbiome data stored in the data storage, thereby performing species identification on the microorganisms in the oral cavity and generating genetic information (S120).

Thereafter, the user terminal 200 transmits the generated genetic information about the subject to the health-care server 300 (S130), and the health-care server 300 classifies and stores genetic information per subject (S140) and obtains the species and population of the microorganisms possessed by the subject through genetic analysis (S150).

Further, the health-care server 300 quantifies the risk level of development of disease for the subject according to the obtained species and population of the microorganisms to thereby obtain a disease score, and generates health information to which the disease score is applied (S150).

Next, the health-care server 300 provides the generated health information to the subject's user terminal 200 (S160). Thus, the user may recognize his current health status through the health information and predict and prepare for disease that he may have.

Meanwhile, after step S160, the health-care server 300 may compare health information, accumulated for a predetermined period, with the current health information to thereby predict each subject's current health status and future health status and apply the health status to the health information or may extract health information about a plurality of other users than any one subject and derive relative health statuses through comparison with the health information about the current subject and provide the relative health statuses to the corresponding user terminal.

The technical spirit of the present invention is described with reference to an example of a screen provided by a user terminal of a health prediction system according to an embodiment of the present invention.

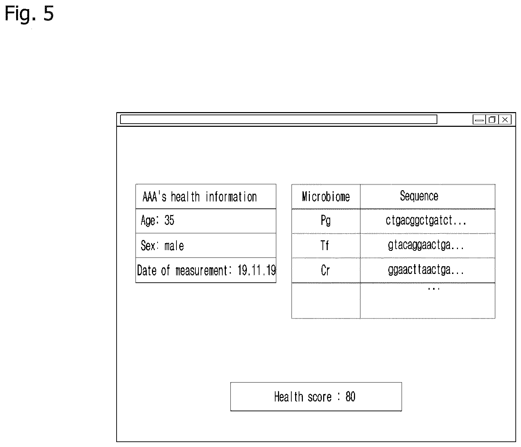

FIG. 5 is a view illustrating an example of a screen provided by a health prediction system using an intraoral microorganism analysis device according to an embodiment of the present invention.

Referring to FIG. 5, the health prediction system using an intraoral microorganism analysis device according to an embodiment of the present invention analyzes a sample collected from the subject through the genome analyzer to thereby generate genetic information, provides the genetic information to the health-care server to thereby perform genetic analysis, and receives health information which is the result of the analysis.

Accordingly, the user terminal may display the subject's health information on the screen. The health information may include the subject's name, gender, or other personal information, and the health score according to the disease risk level calculated based on the type and population of microorganisms, which is displayed as a numerical value.

Further, the health information may further include the genetic sequences for the microorganisms species-identified, as reference information.

The foregoing specific description should be interpreted as an example of a preferred embodiment, rather than as limiting the scope of the present invention. Accordingly, the scope of the present invention should be defined by the following claims and equivalents thereof, but not by the above-described embodiments.

## Claims

1. A health prediction system using an intraoral microorganism analysis device, comprising:
at least one microorganism analysis devices analyzing a sample collected from a subject's oral cavity, performing species identification on the sample, and generating genetic information about a microorganism in the oral cavity according to a result of analysis; and
a health-care server collecting the genetic information for each subject from the microorganism analysis device, analyzing the genetic information to obtain a type and population for one or more microorganisms present in the subject's oral cavity, and generating health information about the subject based on a result of the obtaining the type and population.

2. The health prediction system of claim 1, wherein the microorganism analysis device includes:
a genome analyzer performing genome analysis on the sample; and
a user terminal connected with the genome analyzer, receiving a result of the genome analysis, and performing species identification on the microorganism in the oral cavity through comparison with previously stored microbiome data.

3. The health prediction system of claim 2, wherein the user terminal includes:
a device connector wiredly or wirelessly connected with the genome analyzer to receive the genetic information;
a communication unit connected with the health-care server through an information communication network to perform data communication;
data storage storing microbiome data for a plurality of microorganisms for species identification;
a genetic information transmitter providing the health-care server with latest genetic information about the subject, generated in response to the result of analysis received from the genome analyzer; and
a species identifier performing species identification on one or more microorganisms possessed by the subject through comparison between the result of analysis and the microbiome data and determining the population.

4. The health prediction system of claim 3, wherein the user terminal includes a health information receiver receiving a health score represented as a numerical value for the subject's health status, from the health-care server through the communication unit.

5. The health prediction system of claim 1, wherein the health-care server includes:
a data collector collecting genetic information from one or more user terminals located in areas, through an information communication network;
a member information manager performing a membership sign-up, login, and identification procedure for one or more subjects having membership and providing functions of generating, updating, and deleting member information about the subject;
a database classifying and storing the member information, genetic information for each subject, and health information and deleting or masking genetic information that has passed a predetermined time after the health information about the subject is generated;
a gene analyzer determining the type and population of the microorganism possessed by the subject through the genetic information and estimating and quantifying the subject's current health status based on the type and population of the microorganism to thereby generate the health information;
a report generator converting accumulated information about various microbiomes into a report form; and
a result provider providing the health information about the subject to each user terminal through the information communication network.

6. The health prediction system of claim 5, wherein the health-care server includes a predictor predicting each subject's current health status and future health status by comparing the health information accumulated for a predetermined period with the current health information and applying the predicted health statuses to the health information.

7. The health prediction system of claim 5, wherein the result provider derives a relative health status between health information about any one subject and health information about a plurality of other subjects and further provides the relative health status to the user terminal.

8. A health prediction method using a microorganism analysis device in an oral cavity, comprising:
transmitting a result of genome analysis on a sample collected from a subject to a user terminal, by a genome analyzer;
generating genetic information by performing species identification on a microorganism in the oral cavity according to the result of genome analysis, by the user terminal;
transmitting the genetic information to a health-care server, by the user terminal;
classifying genetic information per subject and obtaining a type and population of microorganism possessed by the subject through genetic analysis to generate health information, by the health-care server; and
providing the health information to the user terminal, by the health-care server.

9. The health prediction method of claim 8, further comprising, after, providing the health information to the user terminal by the health-care server, predicting each subject's current health status and future health status by comparing the health information accumulated for a predetermined period with the current health information and applying the predicted health statuses to the health information, by the health-care server.

10. The health prediction method of claim 8, further comprising, after, providing the health information to the user terminal by the health-care server, extracting health information about a plurality of other users than any one subject, deriving a relative health status through comparison with the subject's current health information, and providing the relative health status to the user terminal, by the health-care server.
